# EUROPEAN PATENT SPECIFICATION

(11) **EP 1 539 115 B1**
(45) Date of publication and mention of the grant of the patent: **19.09.2007**
(21) Application number: 03771910.1
(22) Date of filing: 28.07.2003
(51) Int. Cl.: A61K 9/24, A61K 31/506, A61P 25/18

(54) **METHODS AND DOSAGE FORMS FOR CONTROLLED DELIVERY OF PALIPERIDONE**
VERFAHREN UND DOSIERFORMEN FÜR DIE KONTROLLIERTE ABGABE VON PALIPERIDON
PROCÉDÉ ET FORMULATIONS POUR LA LIBÉRATION CONTRÔLÉE DE PALIPERIDONE

(30) Priority: 29.07.2002 US 399590 P; 26.08.2002 US 406005 P
(43) Date of publication of application: 15.06.2005
(73) Proprietor: ALZA CORPORATION, Mountain View, CA 94039-7210 (US)
(72) Inventor: YAM, Noymi, V., Sunnyvale, CA 94086 (US); REYES, Iran, San Jose, CA 95124 (US); DAVAR, Nipun, Fremont, CA 94555 (US); AYER, Atul, D., Palo Alto, CA 94303 (US); LEE, Julie, Sunnyvale, CA 94086 (US)
(74) Representative: Williams, Paul Edwin
(86) International application number: PCT/US2003/023433
(87) International publication number: WO 2004/010981

(56) References cited:
- WO-A-00/35419
- WO-A-97/44039
- WO-A-99/62496
- US-A1- 2002 035 357
- US-A1- 2002 051 807

## Description

### FIELD OF THE INVENTION

This invention pertains to the controlled delivery of pharmaceutical agents, dosage forms and devices. In particular, the invention is directed to, dosage forms and devices for the controlled delivery of paliperidone, with reduced degradation of the active agent.

### BACKGROUND OF THE INVENTION

The art is replete with descriptions of oral dosage forms for the controlled release of pharmaceutical agents. While a variety of sustained release dosage forms for delivering certain drugs exhibiting short half-life may be known, not every drug may be suitably delivered from those dosage forms because of solubility, metabolic processes, absorption and other physical, chemical and physiological parameters that may be unique to the drug and the mode of delivery. Examples of such drugs that are not likely candidates for controlled release dosage forms are those exhibiting a long half-life such as paliperidone. It has also been found that paliperidone degrades into notable amounts of impurities. The major degradation products include C-9 ketone, N-oxides, and various dimmers of its degradants.

Paliperidone is more fully described in US Pat. No. 4,804,663. The paliperidone compound differs from risperidone and related prior art compounds described in US Pat. Nos. 4,352,811 and 4,458,076 by its substitution on the 1-position of the piperidine moiety.

Paliperidone is practically insoluble in water, freely soluble in methylene chloride and soluble in methanol and 0.1 N hydrochloric acid. Additionally, since paliperidone has a long half-life of about one day, it is not a typical candidate for extended delivery. However, side effects such as anxiety, somnolence, dizziness, constipation, extrapyramidal symptoms, may be related to high blood plasma concentration levels restricting the ability to administer a single daily immediate release dose.

Traditional stability improvements for pharmaceutical agents were explored including use of antioxidants (increased levels of BHT), and incorporation of chelating agents. These traditional methods for reducing degradation proved insufficient.

It is expected that the side effects are likely a result of either rate of rise and/or actual drug blood plasma concentrations exceeding a threshold maximum tolerable concentration (MTC). However, in order to obtain a therapeutic effect, concentrations need to be sustained above a minimum pharmacodynamic concentration (MPC).

Another aspect of delivery of paliperidone is that administration may require low drug loading in the dosage form. Dosage forms may need to contain drug in the range of 5% to 20% of the overall weight of the dosage form. The low drug loading requirement presents problems in formulating compositions and fabricating dosage forms that are suitable for oral administration that deliver at the desired rate of release for an extended period of time.

Prior art osmotic dosage forms mention delivery of risperidone from a liquid gelatin capsule without mention of delivery of paliperidone or of a preferred rate of delivery or identification of a solid capsule dosage form. Published patent application by ALZA Corporation, WO 00/35419.

Other art discloses delivery of risperidone through transdermal methods with patches without claiming any rate of release or desired plasma concentration profile. Published patent application by Janssen, WO 96/31201. Furthermore, this art does not identify delivery of paliperidone much less delivery of paliperidone through oral controlled release delivery.

There is also art disclosing delivery of risperidone and/or paliperidone through injectable implants for long term, multi-day, delivery. This art includes the published patent application by Alkermes WO 01/34120, and US Pat. Nos. 5,654,008; 5650,173; 5,770,231; 6,077,843; 6,368,632; 6,110,923; 5,965,168; and 5,692,477 by Alkermes. US patents claiming injectable dosage forms to provide almost zero order delivery include US Pat. Nos. 5,871,778 and 5,656,299 by Yoishitomi Pharmaceutical Industries. This art does not disclose preferred release rates and does not teach or motivate toward an ascending rate of release, much less such release through an oral delivery system.

Prior art for oral delivery does not address delivery of extended, controlled release paliperidone.

Oral controlled release dosage forms include, US Pat. No. 5,536,507 which describes a three component pharmaceutical formulation that utilizes, *inter alia,* a pH sensitive polymer and optionally an osmotic agent that will swell in the higher pH regions of the lower portion of the small intestine and the large intestine to release drug in those environments. Additional components of the dosage form include a delayed release coating and an enteric coating to provide a dosage form that releases very little, if any, of the drug in the stomach, a relatively minimal amount in the small intestine and reportedly about 85% or more in the large intestine. Such a dosage form provides for a widely varying time-release of drug after administration that may not begin for 1-3 hours until the dosage form has passed from the stomach and an additional 3 hours or more for the dosage form to pass into the large intestine.

Exemplary sustained release paliperidone dosage forms, methods of preparing such dosage forms and methods of using such dosage forms described herein are directed to osmotic dosage forms for oral administration.

In addition to osmotic systems as described herein, however, there are many other approaches to achieving sustained release of drugs from oral dosage forms known in the art. These different approaches include, for example, diffusion systems such as reservoir devices and matrix devices, dissolution systems such as encapsulated dissolution systems (including, for example, "tiny time pills") and matrix dissolution systems, combination diffusion/dissolution systems and ion-exchange resin systems as described in Remington's Pharmaceutical Sciences, 1990 ed., pp. 1682-1685. Paliperidone dosage forms that operate in accord with these other approaches are encompassed by the scope of the disclosure herein to the extent that the drug release characteristics and/or the blood plasma paliperidone concentration characteristics as recited herein and in the claims describe those dosage forms either literally or equivalently.

Osmotic dosage forms in general utilize osmotic pressure to generate a driving force for imbibing fluid into a compartment formed, at least in part, by a semipermeable membrane that permits free diffusion of fluid but not drug or osmotic agent(s), if present. A significant advantage to osmotic systems is that operation is pH-independent and thus continues at the osmotically determined rate throughout an extended time period even as the dosage form transits the gastrointestinal tract and encounters differing microenvironments having significantly different pH values. A review of such dosage forms is found in Santus and Baker, "Osmotic drug delivery: a review of the patent literature," Journal of Controlled Release 35 (1995) 1-21, incorporated in its entirety by reference herein. In particular, the following U.S. Patents, owned by the assignee of the present application, ALZA Corporation, directed to osmotic dosage forms, are each incorporated in their entirety herein: Nos. 3,845,770; 3,916,899; 3,995,631; 4,008,719; 4,111,202; 4,160,020; 4,327,725; 4,519,801; 4,578,075; 4,681,583; 5,019,397; and 5,156,850.

Devices in which a drug composition is delivered as a slurry, suspension or solution from a small exit orifice by the action of an expandable layer are described in U. S. Patents Nos. 5,633,011; 5,190,765; 5,252,338; 5,620,705; 4,931,285; 5,006,346; 5,024,842; and 5,160,743, which are incorporated herein by reference. Typical devices include an expandable push layer and a drug layer surrounded by a semipermeable membrane. In certain instances, the drug layer is provided with a subcoat to delay release of the drug composition to the environment of use or to form an annealed coating in conjunction with the semipermeable membrane.

Devices in which a drug composition is delivered in a dry state from a large exit orifice by the action of an expandable layer are described in US Patent Nos. 4,892,778, 4,915,949 and 4,940,465. Those references describe a dispenser for delivering a beneficial agent to an environment of use that includes a semipermeable wall containing a layer of expandable material that pushes a dry drug layer out of the compartment formed by the wall. The exit orifice in the device is substantially the same diameter as the inner diameter of the compartment formed by the wall.

While dosage forms delivering the drug composition to the environment of use in the dry state may provide suitable release of drug at various drug loadings over a prolonged period of time, the exposure of the drug layer to the environment of use may result in agitation-dependent release of drug that in some circumstances is difficult to control. Accordingly, it may be advantageous to release the drug as a slurry or suspension that may be metered by control of rate of expansion of the push layer and the size of the exit orifice in the dosage form as in accordance with this invention.

US Patent 5,169,638 describes a buoyant controlled release pharmaceutical powder formulation to be filled into capsules that uses a pH dependent polymer formed from alginic acid and hydroxypropylmethyl cellulose to release pharmaceuticals at a controlled rate. It appears that this capsule formulation was intended to mimic the characteristics of a tableted formulation.

No description is provided of a formulation that provides the uniform release characteristics of the dosage forms containing paliperidone and related compounds of the present invention.

US Patent Nos. 4,892,778 and 4,940,465, describe a dispenser for delivering a beneficial agent to an environment of use that includes a semipermeable wall containing a layer of expandable material that pushes a drug layer out of the compartment formed by the wall. The exit orifice in the device is substantially the same diameter as the inner diameter of the compartment formed by the wall.

US Patent No. 4,915,949, describes a dispenser for delivering a beneficial agent to an environment of use that includes a semipermeable wall containing a layer of expandable material that pushes a drug layer out of the compartment formed by the wall. The drug layer contains discrete tiny pills dispersed in a carrier. The exit orifice in the device is substantially the same diameter as the inner diameter of the compartment formed by the wall.

US Patent No. 5,126,142, describes a device for delivering an ionophore to livestock that includes a semipermeable housing in which a composition containing the ionophore and a carrier and an expandable hydrophilic layer is located, along with an additional element that imparts sufficient density to the device to retain it in the rumen-reticular sac of a ruminant animal. The ionophore and carrier are present in a dry state during storage and the composition changes to a dispensable, fluid-like state when it is in contact with the fluid environment of use. A number of different exit arrangements are described, including a plurality of holes in the end of the device and a single exit of varying diameter to control the amount of drug released per unit time due to diffusion and osmotic pumping.

Prior to this invention, paliperidone's related compound, risperidone, was administered in conventional forms, such as a nonrate-controlling, dose-dumping immediate release tablet, or by a dose-dumping capsule, and usually at multiple, repetitive dosing intervals throughout the day. The product is marketed as Risperdal^{®} by Janssen Pharmaceutica Products, L.P. Physicians' Desk Reference, Thompson Healthcare, 56th Ed., pp. 1796-1800 (2002).

The Risperdal^{®} mode of therapy, however, continues to lead to an initial high dose of risperidone in the blood plasma after administration, followed by a decreased level of risperidone in the blood plasma. Moreover, this peak and trough occurs twice to three times during a 24-hour period due to the multiple dosing regimen. The concentration differences in dosing patterns are related to the presence and absence of administered drug, which is a major disadvantage, associated with this prior dosage form and mode of administration.

Conventional dosage forms and their mode of operation, including dose peaks and valleys, are discussed in Pharmaceutical Sciences, Remington, 18th Ed., pp. 1676-1686 (1990), Mack Publishing Co.; The Pharmaceutical and Clinical Pharmacokinetics, 3rd Ed., pp. 1-28 (1984), Lea and Febreger, Philadelphia; and in U.S. Patents Nos. 3,598,122 and 3,598,123, both issued to Zaffaroni.

A dosage form exhibiting substantially ascending release rate profile is Concerta^{®} marketed by McNeil Consumer Healthcare and ALZA Pharmaceuticals. Physicians' Desk Reference, Thompson Healthcare, 56th Ed., pp. 1998-2001 (2002). The Concerta^{®} product, however indicated for once-a-day administration, only delivers at a substantially ascending rate of release for up to about 8 hours.

Patent applications relating to Concerta^{®} include published PCT Pat. Application No. WO99/62496A1. This patent application discloses the substantially ascending release rate profile related to Concerta^{®} for delivery over about 8 hours for once-a-day dosing.

Related patent applications include published PCT Pat. Application No. WO98/14168; WO98/23263; WO 98/06380A2 and US 2001/0012847A1.

Still other applications relating to providing increasing rate of release delivery profile include US 2002/0035357A1; WO 01/52819A1 and WO 01/37813A2 & A3.

US 2002/0051807A discloses an osmotic device containing controlled release alprazolam in the core optionally in combination with an anti-psychotic agent, in a rapid release external coat.

WO-A-9744039 discloses a pharmaceutical composition suitable as a depot formulation for administration via intramuscular or subcutaneous injection comprising 9-hydroxyrisperidone or derivative thereof and a pharmaceutically acceptable carrier.

There remains a need for effective dosing methods, dosage forms and devices that will permit the controlled release of paliperidone and related compounds over a prolonged period of time at a substantially ascending rate of release to reduce the amount of the active agent that the patient is exposed to at any particular time and to increase the time between dosing, preferably to obtain a once-a-day dosing regimen while reducing associated side effects.

### SUMMARY OF THE INVENTION

The present invention is designed for once-a-day administration of an oral dosage form to deliver paliperidone for more than about 22 hours utilizing a capsule-shaped tablet This approximately 22 hours of release is at a substantially ascending rate of release from the core with 90% delivery occurring at about 20 hours. This novel profile provides therapeutic delivery above the MPC while keeping the plasma levels below the MTC and low enough such that side effects will be reduced and the development of tolerance is increased. This delivery profile provides 24 hours of efficacy without high plasma levels.

The present invention provides for a substantially ascending release rate. It has been surprisingly discovered that the instant profile best provides efficacious therapy over 24 hours while potentially reducing negative side effects associated with administration of the drug.

The present invention utilizes a slow, but substantially ascending, rate of release when the dosage form is likely to be in the colonic region of the gastrointestinal (GI) tract. The profile is not previously used to deliver any drug, but is designed to increase the therapeutic index of paliperidone.

It has been surprisingly found that the described ascending release rate can provide for a substantially ascending blood plasma concentration of drug with peak concentration occurring later than about 16 hours after administration. This ascending blood plasma concentration reduces the intraday tolerance effect developed.

It has been further surprisingly discovered that the addition of an osmagent, salt, into the first drug layer, but not in the second drug layer, impacts the delivery profile such that a substantially ascending release rate results.

It has been further surprisingly discovered that maintaining the ratio of the concentration of drug in the first drug layer and the concentration of drug in the second drug layer impacts the delivery profile such that the desired substantially ascending rate of release results.

According to the present invention there is provided a dosage form comprising two or more layers, said two or more layers comprising a first layer and a second layer, said first layer comprises paliperidone, risperidone or a pharmaceutically acceptable salt thereof, said second layer comprises a polymer; an outer wall surrounding said two or more layers; and an orifice in said outer wall.

The dosage form preferably utilizes a semipermeable membrane surrounding a three-layer-core: in these embodiments the first layer is referred to as a first drug layer and contains low amounts of drug and an osmotic agent such as salt, the middle layer referred to as the second drug layer contains higher amounts of drug, excipients and no salt; and the third layer referred to as the push layer contains osmotic agents and no drug. At least one orifice is drilled through the membrane on the first drug layer end of the capsule-shaped tablet.

In the aqueous environment of the Gl tract, water is imbibed through the semipermeable membrane at a controlled rate determined by the properties of the membrane and the osmolality of the core consitituents. This causes the push layer to swell and the drug layers to hydrate and form viscous, but deformable, masses. The push layer expands against the second drug layer, which in turn pushes against the hydrated first drug layer. The first drug layer, followed by the second drug layer, exits the system through the orifice(s) in the membrane at the same rate that water is imbibed into the core. The biologically inert components of the tablet remain intact during the GI transit and are eliminated as a shell along with insoluble core components.

The dosage form incorporating the present invention is designed to be a once-a-day dosage form that is therapeutically effective while providing increased stability.

The dosage form may comprise a membrane defining a compartment, the membrane surrounding an inner protective subcoat, at least one exit orifice formed or formable therein and at least a portion of the membrane being semipermeable; an expandable layer located within the compartment remote from the exit orifice and in fluid communication with the semipermeable portion of the membrane; a first drug layer located adjacent the exit orifice; and a second drug layer located within the compartment between the first drug layer and the expandable layer, the drug layers comprising the compound paliperidone or a pharmaceutically acceptable acid addition salt thereof.

As the drug is relatively insoluble, the first drug layer has a tendency not to mix into the second drug layer. Depending upon the relative viscosity of the first drug layer and second drug layer, different release profiles are obtained. It is imperative to identify the optimum viscosity for each layer. In the present invention, viscosity is modulated by addition of salt, sodium chloride.

The delivery profile from the core is dependent on the weight, formulation and thickness of each of the drug layers.

The ratio of core diameter to core length is also an important factor. The shape of the system as a capsule shaped tablet is an important feature contributing to the substantially ascending profile from the core.

The delivery system is designed to achieve maximum blood plasma concentrations between 14 and 22 hours and preferably between 18 and 21 hours after dosing. Peak concentrations most preferably occur between approximately hour 18 and hour 20.

The present invention is designed to be a once-a-day dosage form that is therapeutically effective while producing fewer side effects than an immediate release dosage form administered multiple times per day. The present invention provides two key features: a substantially ascending delivery that affects the pharmacodynamics and development of tolerance, and the substantially ascending delivery provides adequate plasma concentrations for pharmacological effect. Development of tolerance is related to the sedation effects (using a representative measure such as digit vigilance).

In one aspect, the invention provides a sustained release dosage form adapted to release over a prolonged period of time at a substantially ascending rate of release, the compound paliperidone.

The present invention also provides use of a pharmaceutically effective amount of paliperidone, risperidone or a pharmaceutically acceptable salt thereof in the manufacture of the above-described dosage form for reducing the side effects associated with high blood plasma concentration of antipsychotic agents, wherein said dosage form maintains an ascending release rate of said poliperidone, risperidone or pharmaceutically acceptable salt thereof over a prolonged period of time. Most preferably, the dosage form is administered orally, once a day.

The dosage form of the invention may comprise a membrane defining a compartment, the membrane having at least one exit orifice formed or formable therein and at least a portion of the membrane being semipermeable; an expandable layer located within the compartment remote from the exit orifice and in fluid communication with the semipermeable, portion of the membrane; a first drug layer located adjacent the exit orifice; and a second drug layer located within the compartment between the first drug layer and the expandable layer, the drug layers comprising the compound paliperidone or a pharmaceutically acceptable acid addition salt thereof.

The dosage form of the invention may comprise a membrane defining a compartment, the membrane having at least one exit orifice formed or formable therein and at least a portion of the membrane being semipermeable; an expandable layer located within the compartment remote from the exit orifice and in fluid communication with the semipermeable portion of the membrane; a first drug layer located adjacent the exit orifice; and a second drug layer located within the compartment between the first drug layer and the expandable layer; the drug layers comprising the compound paliperidone or a pharmaceutically acceptable acid addition salt thereof, and the first drug layer comprising salt and the second drug layer containing no salt.

The dosage form may optionally comprise a flow-promoting layer between the membrane and the drug layers.

The invention may be used to treat a condition responsive to administration of paliperidone or a pharmaceutically acceptable acid addition salt thereof, by administering the compound to provide a substantially ascending plasma concentration of the compound. The Cₘₐₓ occurs at a time greater than about 16 hours and preferably at about 20 hours.

### BRIEF DESCRIPTION OF THE FIGURES

Figure 1 illustrates a two-orifice embodiment of the present invention, prior to administration to a subject;

Figure 2 illustrates the model delivery profile providing a substantially ascending rate of release of paliperidone demonstrating the effect of different types of lubricating subcoats;

Figure 3 illustrates an embodiment of the present invention prior to administration to a subject, with an optional lubricating subcoat and barrier layer;

Figure 4 illustrates the model delivery profile providing a substantially ascending rate of release of paliperidone demonstrating the effect of different amounts of sodium chloride in the first drug layer;

Figure 5 illustrates the effect of drug concentration ratio between the first drug layer and the second drug layer on rate of release of paliperidone;

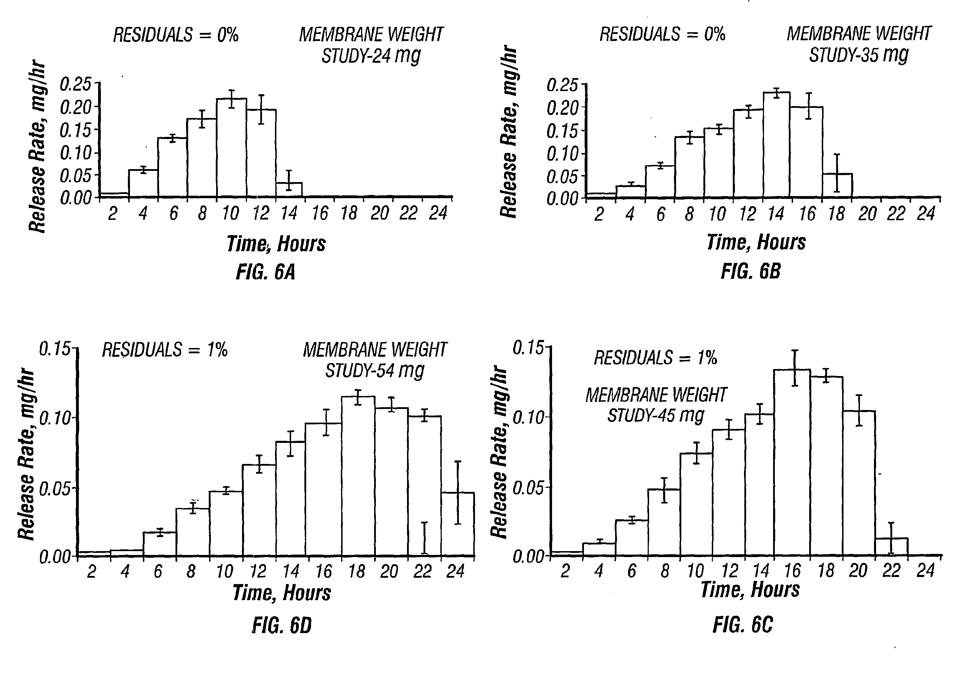

Figures 6A, 6B, 6C and 6D illustrate the effect of membrane weight on rate of release of paliperidone;

Figure 7 illustrates potential degradation pathways for paliperidone under stress conditions;

Figure 8 illustrates tabular comparison of dosage form stability with and without use of the protective subcoat of the present invention;

Figures 9A and 9B illustrate graphical comparison of dosage form stability with and without use of the protective subcoat of the present invention;

### DETAILED DESCRIPTION OF THE INVENTION

The present invention is best understood by reference to the following definitions, the drawings and exemplary disclosure provided herein.

### Definitions

By "dosage form" is meant a pharmaceutical composition or device comprising a pharmaceutically active agent, such as paliperidone or a pharmaceutically acceptable acid addition salt thereof, the composition or device optionally containing inactive ingredients, i.e., pharmaceutically acceptable excipients such as suspending agents, surfactants, disintegrants, binders, diluents, lubricants, stabilizers, antioxidants, osmotic agents, colorants, plasticizers, coatings and the like, that are used to manufacture and deliver active pharmaceutical agents.

By "active agent", "drug", or "compound" is meant an agent, drug, or compound having the characteristics of paliperidone or risperidone or a pharmaceutically acceptable acid addition salt thereof.

By "pharmaceutically-acceptable acid addition salt" or "pharmaceutically acceptable salt", which are used interchangeably herein, are meant those salts in which the anion does not contribute significantly to the toxicity or pharmacological activity of the salt, and, as such, they are the pharmacological equivalents of the bases of the paliperidone compound. Examples of pharmaceutically acceptable acids that are useful for the purposes of salt formation include but are not limited to hydrochloric, hydrobromic, hydroiodic, citric, acetic, benzoic, mandelic, phosphoric, nitric, mucic, isethionic, palmitic, and others.

The expressions "exit," "exit orifice," "delivery orifice" or "drug delivery orifice," and other similar expressions, as may be used herein include a member selected from the group consisting of a passageway; an aperture; an orifice; and a bore. The expression also includes an orifice that is formed or formable from a substance or polymer that erodes, dissolves or is leached from the outer wall to thereby form an exit orifice. The expression includes one or multiple passageways, apertures, orifices, bores or pores.

A drug "release rate" refers to the quantity of drug released from a dosage form per unit time, e.g., milligrams of drug released per hour (mg/hr). Drug release rates for drug dosage forms are typically measured as an *in vitro* rate of dissolution, i.e., a quantity of drug released from the dosage form per unit time measured under appropriate conditions and in a suitable fluid. The dissolution tests described herein were performed on dosage forms placed in metal coil sample holders attached to a USP Type VII bath indexer in a constant temperature water bath at 37°C. Aliquots of the release rate solutions were injected into a chromatographic system to quantify the amounts of drug released during the testing intervals.

By "release rate assay" is meant a standardized assay for the determination of the release rate of a compound from the dosage form tested using a USP Type VII interval release apparatus. It is understood that reagents of equivalent grade may be substituted in the assay in accordance with generally accepted procedures.

For clarity and convenience herein, the convention is utilized of designating the time of drug administration as zero hours (t = 0 hours) and times following administration in appropriate time units, e.g., t = 30 minutes or t = 2 hours, etc.

As used herein, unless otherwise specified, a drug release rate obtained at a specified time "following administration" refers to the *in vitro* drug release rate obtained at the specified time following implementation of an appropriate dissolution test. The time at which a specified percentage of the drug within a dosage form has been released may be referenced as the "Tₓ" value, where "x" is the percent of drug that has been released. For example, a commonly used reference measurement for evaluating drug release from dosage forms is the time at which 70% or 90% of drug within the dosage form has been released. This measurement is referred to as the "T₇₀" or "T₉₀" for the dosage form.

By "immediate-release dosage form" is meant a dosage form that releases drug substantially completely within a short time period following administration, i.e., generally within a few minutes to about 1 hour.

By "extended release dosage form" or "controlled release dosage form" is meant a dosage form that releases drug in a substantially consistent predetermined rate for many hours. Controlled release dosage forms in accord with the present invention exhibit T₉₀ values of at least about 18 hours or more and preferably about 20 hours or more. The dosage forms release drug over periods of time of at least about 16 hours, preferably 18 hours or more and, more preferably, 20 hours or more.

By "sustained release dosage form" is meant a dosage form that releases drug substantially continuously for many hours. Sustained release dosage forms of the present invention release drug over periods of time of at least about 16 hours, preferably about 20 hours or more and, more preferably, about 20 hours or more.

Dosage forms in accord with the present invention exhibit controlled release rates of paliperidone for a prolonged period of time.

By "sustained release " is meant a predetermined continuous release of active agent to an environment over a prolonged period of time.

By "uniform release rate" is meant an average hourly release rate from the core that varies positively or negatively by no more than about 30% and preferably no more than about 25%, most preferably no more than about 10%, from either the preceding or the subsequent average hourly release rate as determined in a USP Type VII Interval Release Apparatus where the cumulative release is between 25% and 75%.

By "prolonged period of time" is meant a continuous period of time of at least about 8 hours, preferably 10-14 hours or more and, more preferably, 16 hours or more. For example, the exemplary osmotic dosage forms described herein generally begin releasing paliperidone at about one hour following administration and the uniform rate of release, as defined above, continues for a prolonged period of time from about 25% to until at least about 75% and preferably at least about 85% of the drug is released from the dosage form. Release of paliperidone continues thereafter for several more hours although the rate of release is generally slowed somewhat from the uniform release rate.

By "C" is meant the concentration of drug in the blood plasma of a subject, generally expressed as mass per unit volume, typically nanograms per milliliter. For convenience, this concentration may be referred to as "plasma drug concentration" or "plasma concentration" herein which is intended to be inclusive of drug concentration measured in any appropriate body fluid or tissue. The plasma drug concentration at any time following drug administration is referenced as Cₜᵢₘₑ, as in C₉ₕ or C₂₄ₕ, etc.

By "steady state" is meant the condition in which the amount of drug present in the blood plasma of a subject does not vary significantly over a prolonged period of time. A pattern of drug accumulation following continuous administration of a constant dose and dosage form at constant dosing intervals eventually achieves a "steady-state" where the plasma concentration peaks and plasma concentration troughs are essentially identical within each dosing interval. As used herein, the steady-state maximal (peak) plasma drug concentration is referenced as Cₘₐₓ and the minimal (trough) plasma drug concentration is referenced as Cₘᵢₙ. The times following drug administration at which the steady-state peak plasma and trough drug concentrations occur are referenced as the Tₘₐₓ and the Tₘᵢₙ, respectively.

Persons of skill in the art appreciate that plasma drug concentrations obtained in individual subjects will vary due to intrapatient variability in the many parameters affecting drug absorption, distribution, metabolism and excretion. For this reason, unless otherwise indicated, mean values obtained from groups of subjects are used herein for purposes of comparing plasma drug concentration data and for analyzing relationships between *in vitro* dosage form dissolution rates and *in vivo* plasma drug concentrations.

A relationship between an administered dose of paliperidone and the magnitude of the peak plasma paliperidone concentration obtained following dose administration is used herein to illustrate significant differences between the dosage forms and methods of the present invention and prior art dosage forms. For example, as described below in more detail, a unitless numerical value is derived by calculating the ratio of the numerical value of the mean Cₘₐₓ (ng/ml) to the numerical value of the dose (mg), i.e., Cₘₐₓ/dose. The difference in the values of the derived ratios characterize the reduction in the magnitude of peak plasma paliperidone concentrations following administration of the sustained release paliperidone dosage forms of the present invention compared to peak plasma paliperidone concentrations following administration of conventional immediate-release paliperidone dosage forms. Administration of dosage forms in accord with the present invention preferably provides steady-state Cₘₐₓ/dose ratios of less than about 30 and more preferably less than about 25.

It has been surprisingly discovered that sustained release paliperidone dosage forms exhibiting T₉₀ values of about 16 hours or more and more preferably about 20 hours or more and which release paliperidone at a controlled release rate for a prolonged period of time can be prepared. Administration of such dosage forms once daily provides therapeutically effective average steady-state plasma paliperidone concentrations.

The exemplary sustained release paliperidone dosage forms, methods of preparing such dosage forms and methods of using such dosage forms described herein are directed to osmotic dosage forms for oral administration. In addition to osmotic systems as described herein, however, there are many other approaches to achieving sustained release of drugs from oral dosage forms known in the art. These different approaches may include, for example, diffusion systems such as reservoir devices and matrix devices, dissolution systems such as encapsulated dissolution systems (including, for example, "tiny time pills") and matrix dissolution systems, combination diffusion/dissolution systems and ion-exchange resin systems as described in Remington's Pharmaceutical Sciences, 1990 ed., pp. 1682-1685. Paliperidone dosage forms that operate in accord with these other approaches are encompassed by the scope of the claims below to the extent that the drug release characteristics and/or the plasma paliperidone concentration characteristics as recited in the claims describe those dosage forms either literally or equivalently.

Osmotic dosage forms, in general, utilize osmotic pressure to generate a driving force for imbibing fluid into a compartment formed, at least in part, by a semipermeable wall that permits free diffusion of fluid but not drug or osmotic agent(s), if present. A significant advantage to osmotic systems is that operation is pH-independent and thus continues at the osmotically determined rate throughout an extended time period even as the dosage form transits the gastrointestinal tract and encounters differing microenvironments having significantly different pH values. A review of such dosage forms is found in Santus and Baker, "Osmotic drug delivery: a review of the patent literature," Journal of Controlled Release 35 (1995) 1-21. In particular, the following U.S. Patents, owned by the assignee of the present application, ALZA Corporation, directed to osmotic dosage forms, are each incorporated in their entirety herein: Nos. 3,845,770; 3,916,899; 3,995,631; 4,008,719; 4,111,202; 4,160,020; 4,327,725; 4,519,801; 4,578,075; 4,681,583; 5,019,397; and 5,156,850.

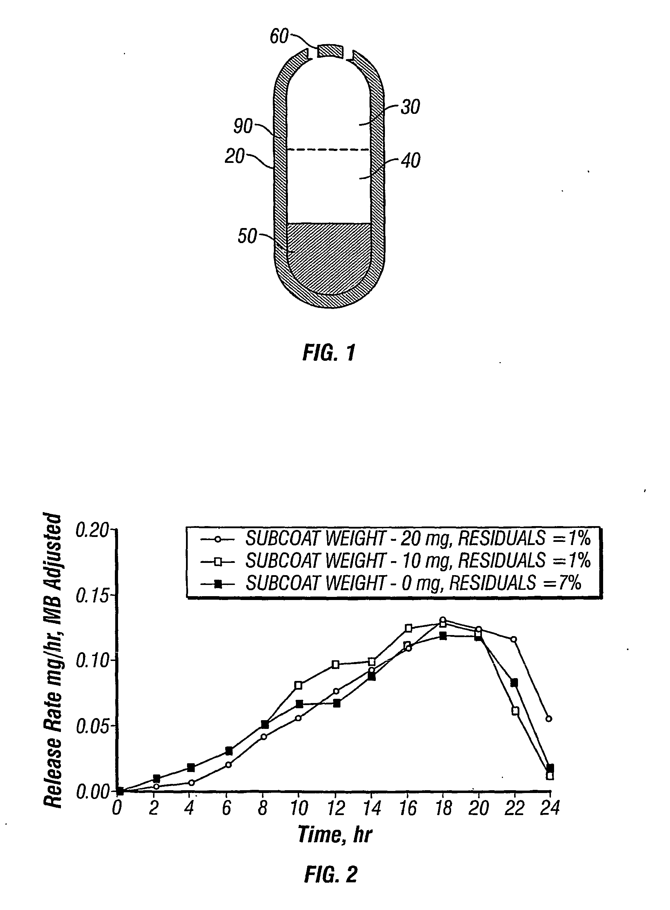

Figure 1 is a cutaway view of one embodiment of dosage form 10 in accord with the present invention. In this embodiment, the internal compartment defined by membrane 20 contains a multilayer-compressed core having a first component drug layer 30, a second component drug layer 40 and a third component push layer 50.

While the preferred embodiment in Figure 1 illustrates a capsule-shaped tablet, the tablet geometry may be other shapes including a standard biconvex shape. Such a preferred shape as well as other alternate shapes will impact and alter release rates.

In operation, following oral ingestion of dosage form 10, the osmotic activity gradient across wall 20 causes gastric fluid to be imbibed through wall 20 thereby converting first drug layer 30 and second drug layer 40 into deliverable compositions, i.e. solutions or suspensions, and concurrently swelling the osmopolymer(s) in push layer 50. The deliverable first drug layer 30 and second drug layer 40 are released through exits 60 as fluid continues to enter the internal compartment and push layer 50 continues to swell. As release of first drug layer 30 and second drug layer 40 occurs, fluid continues to be imbibed and push layer 50 continues to swell thereby driving continued release. In this manner, drug is released in a continuous manner over an extended time period.

As described in more detail below, third component push layer 50 comprises osmotically active component(s), but does not contain active drug. The osmotically active component(s) in push layer 50 typically comprises an osmagent and one or more osmopolymer(s) having relatively large molecular weights which exhibit swelling as fluid is imbibed such that significant release of these osmopolymers through exits 60 does not occur. Additional excipients such as binders, lubricants, antioxidants and colorants may also be included in push layer 50. The third component layer is referred to herein as an expandable or a push layer since, as fluid is imbibed, the osmopolymer(s) swell and push against the deliverable drug formulation of the second component drug layer to thereby facilitate release of the drug formulation from the dosage form.

As described in more detail below, first component drug layer 30 comprises osmotically active components, and a lower amount of active drug than in second component drug layer 40. The osmotically active component(s) in the first component drug layer comprises an osmagent such as salt and one or more osmopolymer(s) having relatively small molecular weights which exhibit swelling as fluid is imbibed such that release of these osmopolymers through exit 60 occurs similar to that of drug layer 40. Additional excipients such as binders, lubricants, antioxidants and colorants may also be included in first drug layer 30.

Second drug layer 40 comprises paliperidone in an admixture with selected excipients adapted to provide an osmotic activity gradient for driving fluid from an external environment through membrane 20 and for forming a deliverable drug formulation upon imbibition of fluid. The excipients may include a suitable suspending agent, also referred to herein as a drug carrier, but no osmotically active agent, "osmagent," such as salt, sodium chloride. It has been surprisingly discovered that the omission of salt from this second drug layer, which contains a higher proportion of the overall drug in the dosage form, in combination with the salt in the first drug layer component, provides an improved ascending rate of release creating a longer duration of ascending rate.

Drug layer 40 has a higher concentration of the drug than does drug layer 30. The ratio of the concentration of drug in the first drug layer 30 to the concentration of drug in the second drug layer 40 is maintained at less than 1 and preferably less than 0.33 to provide the desired substantially ascending rate of release.

Drug layer 40 may also comprise other excipients such as lubricants, binders, etc.

Drug layer 40, as with drug layer 30, further comprises a hydrophilic polymer carrier. The hydrophilic polymer provides a particle in the drug composition that contributes to the controlled delivery of the active drug. Representative examples of these polymers are poly(alkylene oxide) of 100,000 to 750,000 number-average molecular weight, including poly(ethylene oxide), poly(methylene oxide), poly(butylene oxide) and poly(hexylene oxide); and a poly(carboxymethylcellulose) of 40,000 to 400,000 number-average molecular weight, represented by poly(alkali carboxymethylcellulose), poly(sodium carboxymethylcellulose), poly(potassium carboxymethylcellulose) and poly(lithium carboxymethylcellulose). Drug layer 40 can further comprise a hydroxypropylalkylcellulose of 9,200 to 125,000 number-average molecular weight for enhancing the delivery properties of the dosage form as represented by hydroxypropylethylcellulose, hydroxypropylmethylcellulose, hydroxypropylbutylcellulose and hydroxypropylpentylcellulose; and a poly(vinylpyrrolidone) of 7,000 to 75,000 number-average molecular weight for enhancing the flow properties of the dosage form. Preferred among these polymers are the poly(ethylene oxide) of 100,000 - 300,000 number average molecular weight. Carriers that erode in the gastric environment, i.e., bioerodible carriers, are especially preferred.

Other carriers that may be incorporated into drug layer 40, and/or drug layer 30, include carbohydrates that exhibit sufficient osmotic activity to be used alone or with other osmagents. Such carbohydrates comprise monosaccharides, disaccharides and polysaccharides. Representative examples include maltodextrins (i.e., glucose polymers produced by the hydrolysis of corn starch) and the sugars comprising lactose, glucose, raffinose, sucrose, mannitol, sorbitol, and the like. Preferred maltodextrins are those having a dextrose equivalence (DE) of 20 or less, preferably with a DE ranging from about 4 to about 20, and often 9-20. Maltodextrin having a DE of 9-12 has been found to be useful.

Drug layer 40 and drug layer 30 typically will be a substantially dry, <1% water by weight, composition formed by compression of the carrier, the drug, and other excipients as one layer.

Drug layer 40 may be formed from particles by comminution that produces the size of the drug and the size of the accompanying polymer used in the fabrication of the drug layer, typically as a core containing the compound, according to the mode and the manner of the invention. The means for producing particles include granulation, spray drying, sieving, lyophilization, crushing, grinding, jet milling, micronizing and chopping to produce the intended micron particle size. The process can be performed by size reduction equipment, such as a micropulverizer mill, a fluid energy grinding mill, a grinding mill, a roller mill, a hammer mill, an attrition mill, a chaser mill, a ball mill, a vibrating ball mill, an impact pulverizer mill, a centrifugal pulverizer, a coarse crusher and a fine crusher. The size of the particle can be ascertained by screening, including a grizzly screen, a flat screen, a vibrating screen, a revolving screen, a shaking screen, an oscillating screen and a reciprocating screen. The processes and equipment for preparing drug and carrier particles are disclosed in Pharmaceutical Sciences, Remington, 17th Ed., pp. 1585-1594 (1985); Chemical Engineers Handbook, Perry, 6th Ed., pp. 21-13 to 21-19 (1984); Journal of Pharmaceutical Sciences, Parrot, Vol. 61, No. 6, pp. 813-829 (1974); and Chemical Engineer, Hixon, pp. 94-103 (1990).

First drug layer 30 comprises paliperidone in an admixture with selected excipients adapted to provide an osmotic activity gradient for driving fluid from an external environment through membrane 20 and for forming a deliverable drug formulation upon imbibition of fluid. The excipients may include a suitable suspending agent, also referred to herein as a drug carrier, and an osmotically active agent, i.e., an "osmagent," such as salt. Other excipients such as lubricants, binders, etc. may also be included. It has been surprisingly found that when first component drug layer 30 comprises an osmotically active component, and a lower amount of active drug than in second component drug layer 40, an improved ascending rate of release can be created that provides a longer duration of ascending rate. Additionally, with the low doses of paliperidone delivered from a dosage form, and the low amount of that total in the first drug layer 30, the addition of salt has been found to provide a consistent predetermined release rate providing a substantially ascending rate of release over 20 hours.

The osmotically active component in the first drug layer typically comprises an osmagent and one or more osmopolymer(s) having relatively small molecular weights which exhibit swelling as fluid is imbibed such that release of these osmopolymers through exit 60 occurs similar to that of drug layer 40.

First drug layer 30 may also comprise additional excipients such as binders, lubricants, antioxidants and colorants.

It has been surprisingly discovered that the ratio of drug concentration between the first drug layer and the second drug layer alters the release rate profile. Release rate profile is calculated as the difference between the maximum release rate and the release rate achieved at the first time point after start-up (for example, at 6 hours), divided by the average release rate between the two data points.

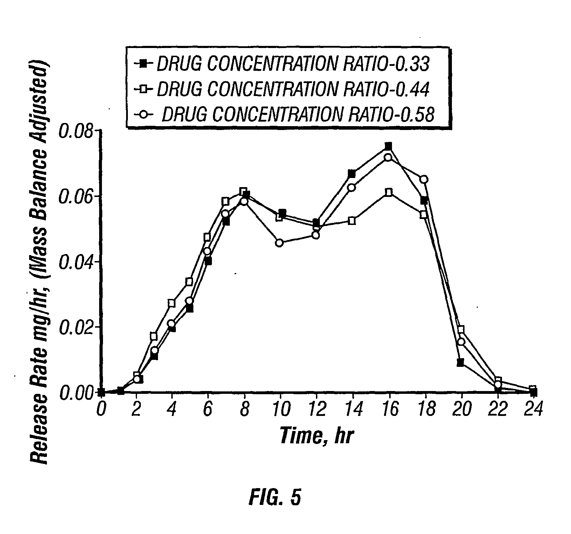

For instance, in Example 1, the drug concentration in first drug layer 30 is 0.8% and the drug concentration in the second drug layer 40 is 2.5% resulting in a 0.33 ratio between the two layers that provides a 60% release rate profile. It has been found that lower drug concentration ratio provide improved release rate curve slope. High drug concentration ratio produces less ascending release rates as shown in Figure 5. The drug concentration ratio to produce greater than 50% ascending release rate curve slope is less than 0.44.

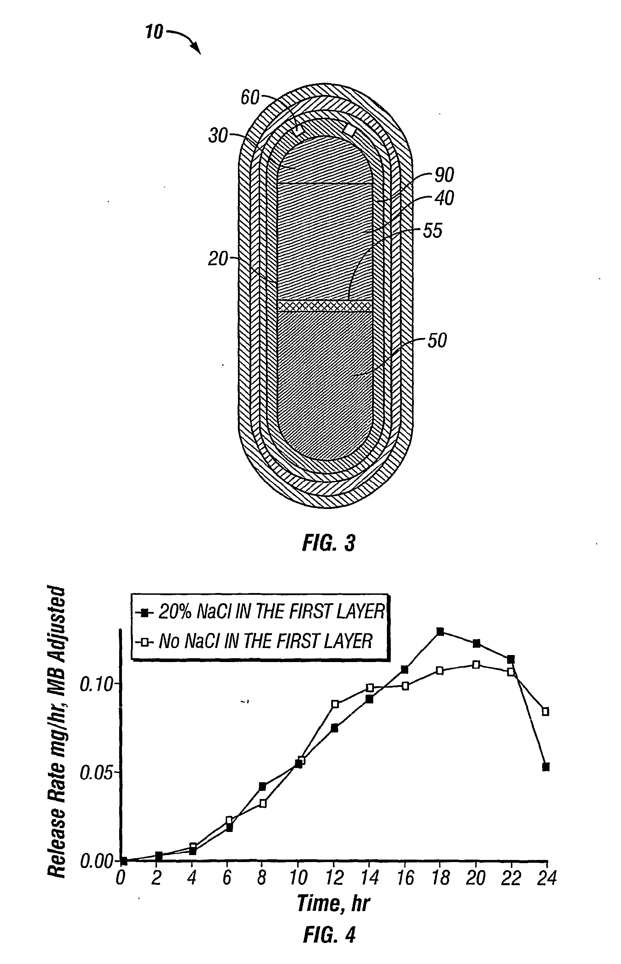

Similarly, it has been found that reduced salt in the first drug layer 30 also reduces the release rate curve slope. For example when no salt is added the release rate curve slope is about 57. When 20% salt is added, the release rate curve slope increases to 80%. See Figure 4. The amount of salt required to provide the preferred ascending release rate profile is at least 20%.

It has been surprisingly discovered that the ascending rate of release of paliperidone provides superior bioavailability, absorption and efficacy over multiple dosings of immediate release dosage forms as well as substantially zero order rates of release over prolonged periods of time.

Drug layer 30 and drug layer 40 may optionally contain surfactants and disintegrants in both drug layers. Exemplary of the surfactants are those having an HLB value of between about 10 - 25, such as polyethylene glycol 400 monostearate, polyoxyethylene-4-sorbitan monolaurate, polyoxyethylene-20-sorbitan monooleate, polyoxyethylene-20-sorbitan monopalmitate, polyoxyethylene-20-monolaurate, polyoxyethylene-40 -stearate, sodium oleate and the like. Disintegrants may be selected from starches, clays, celluloses, algins and gums and crosslinked starches, celluloses and polymers. Representative disintegrants include corn starch, potato starch, croscarmelose, crospovidone, sodium starch glycolate, Veegum HV, methylcellulose, agar, bentonite, carboxymethylcellulose, alginic acid, guar gum and the like.

A representative compound of paliperidone having antipsychotic activity is immediate release risperidone, Risperdal^{®}.

Blood plasma concentrations in a subject may be determined by clinical assay to determine a correlation between tolerability and clinical effect and blood plasma concentrations of drug. The present invention provides for a period of delivery utilizing a substantially ascending blood plasma concentration profile.

Dosage forms of the present invention have core drug release T₉₀ values of greater than 12 hours, preferably greater than 16 hours and most preferably greater than 20 hours, and release paliperidone for a continuous period of time of about 22 hours. After about one hour following administration, the dosage form begins releasing paliperidone from the core at a substantially ascending rate of release that continues for a prolonged period of time of about 16 hours or more.

Wall 20 is formed to be permeable to the passage of an external fluid, such as water and biological fluids, and is substantially impermeable to the passage of paliperidone, osmagent, osmopolymer and the like. As such, it is semipermeable. The selectively semipermeable compositions used for forming wall 20 are essentially nonerodible and substantially insoluble in biological fluids during the life of the dosage form.

Representative polymers for forming wall 20 comprise semipermeable homopolymers, semipermeable copolymers, and the like. Such materials comprise cellulose esters, cellulose ethers and cellulose ester-ethers. The cellulosic polymers have a degree of substitution (DS) of their anhydroglucose unit of from greater than 0 up to 3, inclusive. Degree of substitution (DS) means the average number of hydroxyl groups originally present on the anhydroglucose unit that are replaced by a substituting group or converted into another group. The anhydroglucose unit can be partially or completely substituted with groups such as acyl, alkanoyl, alkenoyl, aroyl, alkyl, alkoxy, halogen, carboalkyl, alkylcarbamate, alkylcarbonate, alkylsulfonate, alkysulfamate, semipermeable polymer forming groups, and the like, wherein the organic moieties contain from one to twelve carbon atoms, and preferably from one to eight carbon atoms.

The semipermeable compositions typically include a member selected from the group consisting of cellulose acylate, cellulose diacylate, cellulose triacylate, cellulose acetate, cellulose diacetate, cellulose triacetate, mono-, di- and tri-cellulose alkanylates, mono-, di-, and tri-alkenylates, mono-, di-, and tri-aroylates, and the like. Exemplary polymers include cellulose acetate having a DS of 1.8 to 2.3 and an acetyl content of 32 to 39.9%; cellulose diacetate having a DS of 1 to 2 and an acetyl content of 21 to 35%; cellulose triacetate having a DS of 2 to 3 and an acetyl content of 34 to 44.8%; and the like. More specific cellulosic polymers include cellulose propionate having a DS of 1.8 and a propionyl content of 38.5%; cellulose acetate propionate having an acetyl content of 1.5 to 7% and an acetyl content of 39 to 42%; cellulose acetate propionate having an acetyl content of 2.5 to 3%, an average propionyl content of 39.2 to 45%, and a hydroxyl content of 2.8 to 5.4%; cellulose acetate butyrate having a DS of 1.8, an acetyl content of 13 to 15%, and a butyryl content of 34 to 39%; cellulose acetate butyrate having an acetyl content of 2 to 29%, a butyryl content of 17 to 53%, and a hydroxyl content of 0.5 to 4.7%; cellulose triacylates having a DS of 2.6 to 3, such as cellulose trivalerate, cellulose trilamate, cellulose tripalmitate, cellulose trioctanoate and cellulose tripropionate; cellulose diesters having a DS of 2.2 to 2.6, such as cellulose disuccinate, cellulose dipalmitate, cellulose dioctanoate, cellulose dicaprylate, and the like; and mixed cellulose esters, such as cellulose acetate valerate, cellulose acetate succinate, cellulose propionate succinate, cellulose acetate octanoate, cellulose valerate palmitate, cellulose acetate heptanoate, and the like. Semipermeable polymers are known in U.S. Patent No. 4,077,407, and they can be synthesized by procedures described in Encyclopedia of Polymer Science and Technology, Vol. 3, pp. 325-354 (1964), Interscience Publishers Inc., New York, NY.

Additional semipermeable polymers for forming wall 20 comprise cellulose acetaldehyde dimethyl acetate; cellulose acetate ethylcarbamate; cellulose acetate methyl carbamate; cellulose dimethylaminoacetate; semipermeable polyamide; semipermeable polyurethanes; semipermeable sulfonated polystyrenes; cross-linked selectively semipermeable polymers formed by the coprecipitation of an anion and a cation, as disclosed in U.S. Patents Nos. 3,173,876; 3,276,586; 3,541,005; 3,541,006 and 3,546,142; semipermeable polymers, as disclosed by Loeb, et al. in U.S. Patent No. 3,133,132; semipermeable polystyrene derivatives; semipermeable poly(sodium styrenesulfonate); semipermeable poly(vinylbenzyltrimethylammonium chloride); and semipermeable polymers exhibiting a fluid permeability of 10⁻⁵ to 10⁻² (cc. mil/cm hr.atm), expressed as per atmosphere of hydrostatic or osmotic pressure differences across a semipermeable wall. The polymers are known to the art in U.S. Patents Nos. 3,845,770; 3,916,899 and 4,160,020; and in Handbook of Common Polymers, Scott and Roff (1971) CRC Press, Cleveland, OH.

Wall 20 may also comprise a flux-regulating agent. The flux regulating agent is a compound added to assist in regulating the fluid permeability or flux through wall 20. The flux-regulating agent can be a flux-enhancing agent or a flux-decreasing agent. The agent can be preselected to increase or decrease the liquid flux. Agents that produce a marked increase in permeability to fluid such as water are often essentially hydrophilic, while those that produce a marked decrease to fluids such as water are essentially hydrophobic. The amount of regulator in the wall when incorporated therein generally is from about 0.01 % to 20% by weight or more. The flux regulator agents may include polyhydric alcohols, polyalkylene glycols, polyalkylenediols, polyesters of alkylene glycols, and the like. Typical flux enhancers include polyethylene glycol 300, 400, 600, 1500, 4000, 6000 and the like; low molecular weight glycols such as polypropylene glycol, polybutylene glycol and polyamylene glycol: the polyalkylenediols such as poly(1,3-propanediol), poly(1,4-butanediol), poly(1,6-hexanediol), and the like; aliphatic diols such as 1,3-butylene glycol, 1,4-pentamethylene glycol, 1,4-hexamethylene glycol, and the like; alkylene triols such as glycerine, 1,2,3-butanetriol, 1,2,4-hexanetriol, 1,3,6-hexanetriol and the like; esters such as ethylene glycol dipropionate, ethylene glycol butyrate, butylene glycol dipropionate, glycerol acetate esters, and the like. Presently preferred flux enhancers include the group of difunctional block-copolymer polyoxyalkylene derivatives of propylene glycol known as pluronics (BASF). Representative flux-decreasing agents include phthalates substituted with an alkyl or alkoxy or with both an alkyl and alkoxy group such as diethyl phthalate, dimethoxyethyl phthalate, dimethyl phthalate, and [di(2-ethylhexyl) phthalate], aryl phthalates such as triphenyl phthalate, and butyl benzyl phthalate; insoluble salts such as calcium sulfate, barium sulfate, calcium phosphate, and the like; insoluble oxides such as titanium oxide; polymers in powder, granule and like form such as polystyrene, polymethylmethacrylate, polycarbonate, and polysulfone; esters such as citric acid esters esterified with long chain alkyl groups; inert and substantially water impermeable fillers; resins compatible with cellulose based wall forming materials, and the like.

Other materials may be included in the semipermeable wall composition for imparting flexibility and elongation properties, for making wall 20 less brittle and to render tear strength. Suitable materials include phthalate plasticizers such as dibenzyl phthalate, dihexyl phthalate, butyl octyl phthalate, straight chain phthalates of six to eleven carbons, di-isononyl phthalte, diisodecyl phthalate, and the like. The plasticizers include nonphthalates such as triacetin, dioctyl azelate, epoxidized tallate, tri-isoctyl trimellitate, tri-isononyl trimellitate, sucrose acetate isobutyrate, epoxidized soybean oil, and the like. The amount of plasticizer in a wall when incorporated therein is about 0.01 % to 20% weight, or higher.

Push layer 50, the third component, comprises an expandable composition in contacting layered arrangement with the second component drug layer 40 as illustrated in Figure 1 or in contacting layered arrangement with barrier layer 55 as illustrated in Figure 3. Push layer 50 comprises a polymer that imbibes an aqueous or biological fluid and swells to push the drug composition through the exit of the device. A polymer having suitable imbibition properties may be referred to herein as an osmopolymer. The osmopolymers are swellable, hydrophilic polymers that interact with water and aqueous biological fluids and swell or expand to a high degree, typically exhibiting a 2-50 fold volume increase. The osmopolymer can be non-crosslinked or crosslinked, but in a preferred embodiment are at least lightly crosslinked to create a polymer network that is too large and entangled to exit the dosage form. Thus, in a preferred embodiment, the expandable composition is retained within the dosage form during its operative lifetime.

Representatives of fluid-imbibing displacement polymers comprise members selected from poly(alkylene oxide) of 1 million to 15 million number-average molecular weight, as represented by poly(ethylene oxide), and poly(alkali carboxymethylcellulose) of 500,000 to 3,500,000 number-average molecular weight, wherein the alkali is sodium, potassium or lithium. Examples of additional polymers for the formulation of the push layer composition comprise osmopolymers that form hydrogels, such as Carbopol^{®} acidic carboxypolymer, a polymer of acrylic cross-linked with a polyallyl sucrose, also known as carboxypolymethylene, and carboxyvinyl polymer having a molecular weight of 250,000 to 4,000,000; Cyanamer^{®} polyacrylamides; cross-linked water swellable indenemaleic anhydride polymers; Good-rite^{®} polyacrylic acid having a molecular weight of 80,000 to 200,000; Aqua-Keeps^{®} acrylate polymer polysaccharides composed of condensed glucose units, such as diester cross-linked polygluran; and the like. Representative polymers that form hydrogels are known to the prior art in U.S. Patent No. 3,865,108, issued to Hartop; U.S. Patent No. 4,002,173, issued to Manning; U.S. Patent No. 4,207,893, issued to Michaels; and in Handbook of Common Polymers, Scott and Roff, Chemical Rubber Co., Cleveland, OH.

Suitable osmagents, also known as osmotic solutes and osmotically effective agents, that may be found in the first drug layer and the push layer in the dosage form are those which exhibit an osmotic activity gradient across the wall 20. Suitable osmagents comprise a member selected from the group consisting of sodium chloride, potassium chloride, lithium chloride, magnesium sulfate, magnesium chloride, potassium sulfate, sodium sulfate, lithium sulfate, potassium acid phosphate, mannitol, urea, inositol, magnesium succinate, tartaric acid, raffinose, sucrose, glucose, lactose, sorbitol, inorganic salts, organic salts and carbohydrates.

Exemplary solvents suitable for manufacturing the dosage form components comprise aqueous or inert organic solvents that do not adversely harm the materials used in the system. The solvents broadly include members selected from the group consisting of aqueous solvents, alcohols, ketones, esters, ethers, aliphatic hydrocarbons, halogenated solvents, cycloaliphatics, aromatics, heterocyclic solvents and mixtures thereof. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride nitroethane, nitropropane tetrachloroethane, ethyl ether, isopropyl ether, cyclohexane, cyclooctane, benzene, toluene, naphtha, 1,4-dioxane, tetrahydrofuran, diglyme, water, aqueous solvents containing inorganic salts such as sodium chloride, calcium chloride, and the like, and mixtures thereof such as acetone and water, acetone and methanol, acetone and ethyl alcohol, methylene dichloride and methanol, and ethylene dichloride and methanol.

Figure 3 illustrates an alternate embodiment including an optional third component barrier layer 55 separating second component drug layer 40 from push layer 50. Figure 3 also illustrates dosage form 10 including an inner wall 90.

Figure 3 illustrates the preferred embodiment of dosage form 10 including the protective inner wall or subcoat 90 and an optional third component barrier layer 55 separating second component drug layer 40 from push layer 50.

The composition of barrier layer 55 is inert with the respect to the composition of second component drug layer 40 and substantially impermeable; such that drug from drug layer 40 and the components of push layer 50 are prevented from mixing. Suitable materials include water-insoluble polymers, fats, fatty acids and fatty acid esters that are solids at ambient and body temperatures, and waxes. Representative water-insoluble polymers include ethyl cellulose, cellulose acetate, polyvinylchloride, copolymers of polyethylene and vinyl acetate, poly(methylmethacrylate), acrylic polymers such as Eudragit^{®} L or Eudragit^{®} R, polycaprolactone, poly(lactic-co-glycolic) acid polymers (PLGA), high density polyethylene, rubber, styrene butadiene, polysilicone, nylon, , polystyrene, polytetrafluoroethylene, and halogenated polymers. Representative waxes include paraffin wax and beeswax. Representative fats, fatty acids and fatty acid esters include C₁₆- C₂₄ long chain fatty acids, esters of such long chain fatty acids such as stearic acid and oleic acid, and mixtures of the foregoing. Mixtures of the above-described materials may be utilized, e.g., a mixture of ethyl cellulose and stearic acid, which is presently preferred.

Protective subcoat 90 is permeable to the passage of gastric fluid entering the compartment defined by wall 20 and provides a protective function that reduces the degradation of paliperidone under stress conditions.

Inner wall 90 further provides a lubricating function that facilitates the movement of first drug layer 30, second drug layer 40 and push layer 50 toward exit 60. Inner wall 90 may be formed from hydrophilic materials and excipients. Outer wall 20 is semipermeable, allowing gastric fluid to enter the compartment, but preventing the passage of the materials comprising the core in the compartment. The deliverable drug formulation is released from exit 60 as described above with respect to the embodiment of Figure 3.

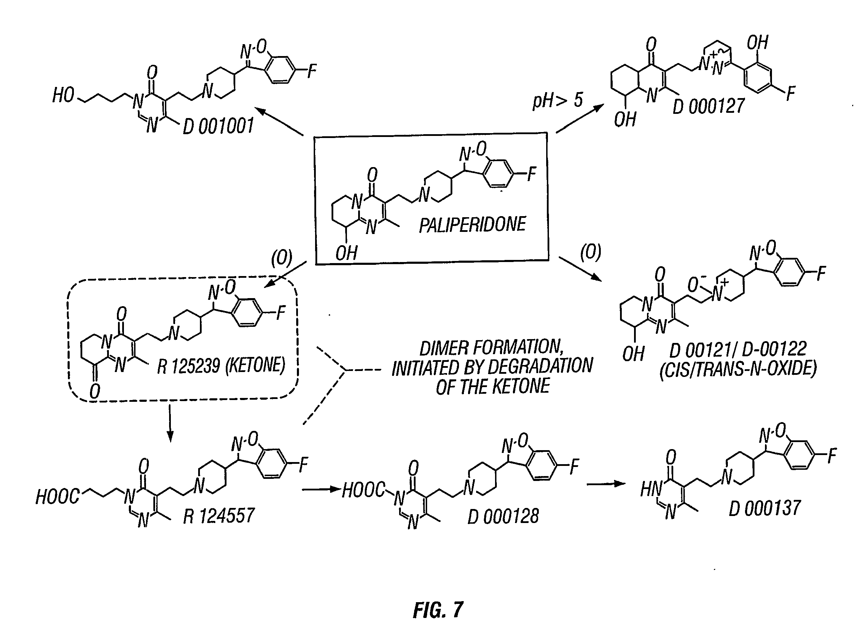

Figure 7 illustrates potential degradation pathways for paliperidone under stress conditions. Subcoat 90 provides a means for reducing this potential degradation.

Inner wall 90 may be formed from hydrophilic materials and excipients. Wall 20 is semipermeable, allowing gastric fluid to enter the compartment, but substantially impermeable to the passage of materials comprising the core in the compartment. The deliverable drug formulation is released from exit 60 as described above with respect to the embodiment of Figure 3.

Inner wall 90, is located between at least drug layers 30 and 40, and wall 20 to reduce degradation of the active agent of drug layer 30 and drug layer 40. Inner wall90 promotes stability of the drug composition.

Inner wall 90 also reduces friction between the external surface of drug layer 30 and drug layer 40, and the inner surface of wall 20. Inner wall 90 promotes release of the drug composition from the compartment and reduces the amount of residual drug composition remaining in the compartment at the end of the delivery period, particularly when the slurry, suspension or solution of the drug composition that is being dispensed is highly viscous during the period of time in which it is being dispensed. In dosage forms in which there is high drug loading, i.e., 40% or greater active agent in the drug layer based on the overall weight of the drug layer, and no inner wall, it has been observed that significant residual amounts of drug may remain in the device after the period of delivery has been completed. In some instances, amounts of 20% or greater may remain in the dosage form at the end of a twenty-four hour period when tested in a release rate assay.

Inner wall 90 is formed as an inner coat of a flow-promoting agent, i.e., an agent that lowers the frictional force between the outer wall 20 and the external surface of drug layer 40. Inner wall 90 appears to reduce the frictional forces between outer wall 20 and the outer surface of drug layer 30 and drug layer 40, thus allowing for more complete delivery of drug from the device. Particularly in the case of active compounds having a high cost, such an improvement presents substantial economic advantages since it is not necessary to load the drug layer with an excess of drug to insure that the minimum amount of drug required will be delivered. Inner wall 90 may be formed as a coating applied over the compressed core.

Inner wall 90 is further characterized by a protective agent, i.e., an agent that reduces the degradation of paliperidone in drug layer 30 and drug layer 40. Particularly in the case of active compounds having a high cost, such an improvement presents substantial economic advantages. Inner wall 90 may be formed as a coating applied over the compressed core.

Inner wall 90 typically may be 0.01 to 5 mm thick, more typically 0.5 to 5mm thick, and it comprises a member selected from hydrogels, gelatin, low molecular weight polyethylene oxides, e.g., less than 100,000 MW, hydroxyalkylcelluloses, e.g., hydroxyethylcellulose, hydroxypropylcellulose, hydroxyisopropylcelluose, hydroxybutylcellulose and hydroxyphenylcellulose, and hydroxyalkyl alkylcelluloses, e.g., hydroxypropyl methylcellulose, and mixtures thereof. The hydroxyalkylcelluloses comprise polymers having a 9,500 to 1,250,000 number-average molecular weight. For example, hydroxypropyl celluloses having number average molecular weights of between 80,000 to 850,000 are useful. The inner wall may be prepared from conventional solutions or suspensions of the aforementioned materials in aqueous solvents or inert organic solvents.

Prefered materials for the inner wall include hydroxypropyl cellulose, hydroxyethyl cellulose, hydroxypropyl methyl cellulose, povidone [poly(vinylpyrrolidone)], polyethylene glycol, and mixtures thereof.

Most prefered are mixtures of hydroxypropyl cellulose and povidone, prepared in organic solvents, particularly organic polar solvents such as lower alkanols having 1-8 carbon atoms, preferably ethanol, mixtures of hydroxyethyl cellolose and hydroxypropyl methyl cellulose prepared in aqueous solution, and mixtures of hydroxyethyl cellulose and polyethylene glycol prepared in aqueous solution. Most preferably, the inner wall comprises a mixture of hydroxypropyl cellulose and providone prepared in ethanol.

It is preferred that inner wall 90 comprises between about 50% and about 90% hydroxypropylcellulose identified as EF having an average molecular weight of about 80,000 and between about 10% and about 50% polyvinylpyrrolidone identified as K29-32.

Conveniently, the weight of the inner wall applied to the compressed core may be correlated with the thickness of the inner wall and residual drug remaining in a dosage form in a release rate assay such as described herein. As such, during manufacturing operations, the thickness of the inner wall may be controlled by controlling the weight of the inner wall taken up in the coating operation.

When inner wall 90 is formed as a subcoat, i.e., by coating onto the tabletted composite including one or all of the first drug layer, second drug layer and push layer, the inner wall can fill in surface irregularities formed on the core by the tabletting process. The resulting smooth external surface facilitates slippage between the coated composite core and the semipermeable wall during dispensing of the drug, resulting in a lower amount of residual drug composition remaining in the device at the end of the dosing period. When inner wall 90 is fabricated of a gel-forming material, contact with water in the environment of use facilitates formation of the gel or gel-like inner coat having a viscosity that may promote and enhance slippage between outer wall 20 and drug layer 30 and drug layer 40.

Subcoat 90 has also been shown to reduce degradation of the paliperidone during stability testing and could improve and extend shelf life of the resulting formulation.

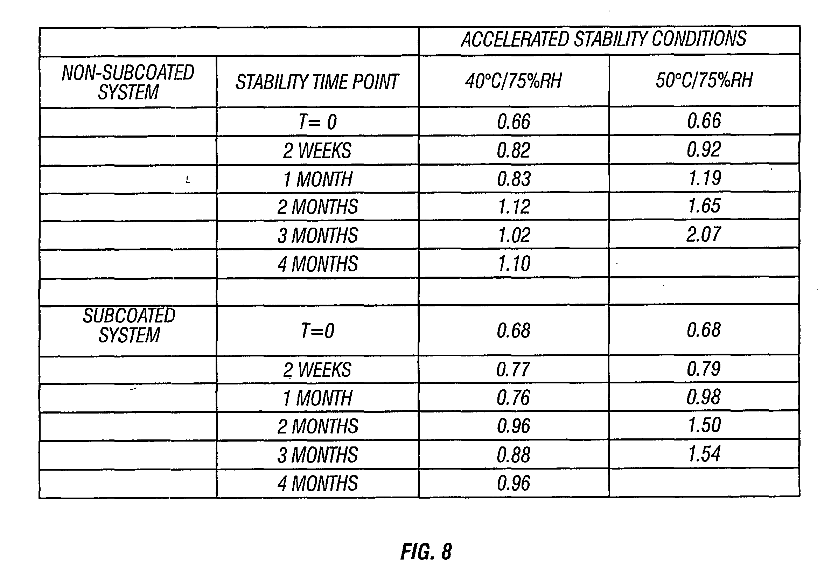

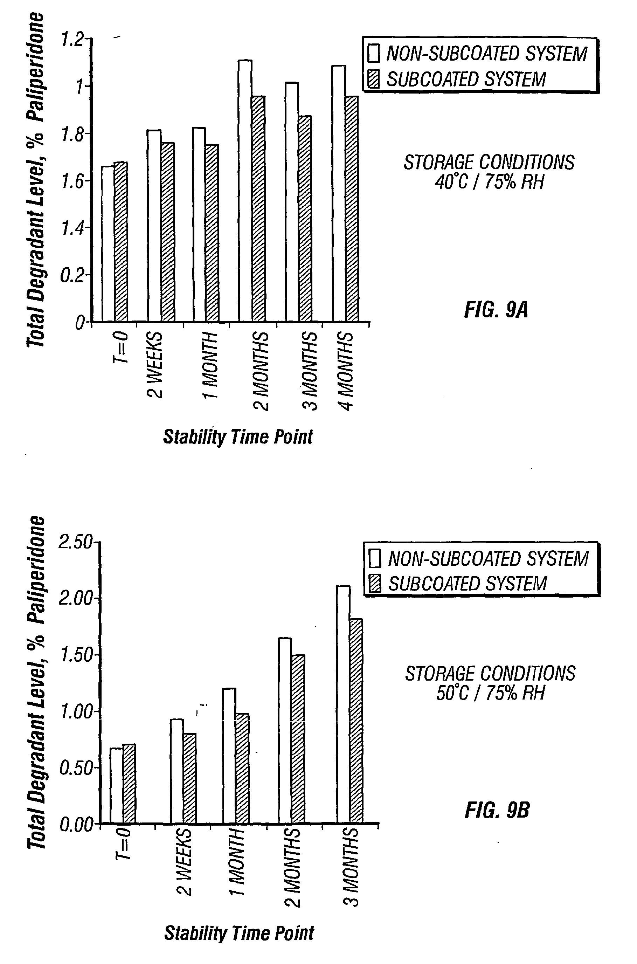

Figures 8, 9A and 9B illustrate the increased stability of paliperidone in the dosage forms incorporating the protective subcoat compared to dosage forms not incorporating the protective subcoat.

Pan coating may be conveniently used to provide the completed dosage form, except for the exit orifice. In the pan coating system, the wall-forming composition for the inner wall or the outer wall, as the case may be, is deposited by successive spraying of the appropriate wall composition onto the compressed trilayered or multilayered core comprising the drug layers, optional barrier layer and push layer, accompanied by tumbling in a rotating pan. A pan coater is used because of its availability at commercial scale. Other techniques can be used for coating the compressed core. Once coated, the wall is dried in a forced-air oven or in a temperature and humidity controlled oven to free the dosage form of solvent(s) used in the manufacturing. Drying conditions will be conventionally chosen on the basis of available equipment, ambient conditions, solvents, coatings, coating thickness, and the like.

Other coating techniques can also be employed. For example, the wall or walls of the dosage form may be formed in one technique using the air-suspension procedure. This procedure consists of suspending and tumbling the compressed core in a current of air and the semipermeable wall forming composition, until the wall is applied to the core. The air-suspension procedure is well suited for independently forming the wall of the dosage form. The air-suspension procedure is described in U.S. Patent No. 2,799,241; in J. Am. Pharm. Assoc., Vol. 48, pp. 451-459 (1959); and, ibid., Vol. 49, pp. 82-84 (1960). The dosage form also can be coated with a Wurster^{®} air-suspension coater using, for example, methylene dichloride methanol as a cosolvent for the wall forming material. An Aeromatic^{®} air-suspension coater can be used employing a cosolvent.

Dosage forms in accord with the present invention are manufactured by standard techniques. For example, the dosage form may be manufactured by the wet granulation technique. In the wet granulation technique, the drug and carrier are blended using an organic solvent, such as denatured anhydrous ethanol, as the granulation fluid. The remaining ingredients can be dissolved in a portion of the granulation fluid, such as the solvent described above, and this latter prepared wet blend is slowly added to the drug blend with continual mixing in the blender. The granulating fluid is added until a wet blend is produced, which wet mass blend is then forced through a predetermined screen onto oven trays. The blend is dried for 18 to 24 hours at 24°C to 35°C in a forced-air oven. The dried granules are then sized. Next, magnesium stearate, or another suitable lubricant, is added to the drug granulation, and the granulation is put into milling jars and mixed on a jar mill for 10 minutes. The composition is pressed into a layer, for example, in a Manesty^{®} press or a Korsch LCT press. For a trilayered core, granules or powders of the drug layer compositions and push layer composition are sequentially placed in an appropriately-sized die with intermediate compression steps being applied to each of the first two layers, followed by a final compression step after the last layer is added to the die to form the trilayered core. The intermediate compression typically takes place under a force of about 50-100 newtons. Final stage compression typically takes place at a force of 3500 newtons or greater, often 3500-5000 newtons. The compressed cores are fed to a dry coater press, e.g., Kilian^{®} Dry Coater press, and subsequently coated with the wall materials as described above.

One or more exit orifices are drilled in the drug layer end of the dosage form, and optional water soluble overcoats, which may be colored (e.g., Opadry colored coatings) or clear (e.g., Opadry Clear), may be coated on the dosage form to provide the finished dosage form.

In another manufacture the drug and other ingredients comprising the drug layer are blended and pressed into a solid layer. The layer possesses dimensions that correspond to the internal dimensions of the area the layer is to occupy in the dosage form, and it also possesses dimensions corresponding to the push layer, if included, for forming a contacting arrangement therewith. The drug and other ingredients can also be blended with a solvent and mixed into a solid or semisolid form by conventional methods, such as ballmilling, calendering, stirring or rollmilling, and then pressed into a preselected shape. Next, if included, a layer of osmopolymer composition is placed in contact with the layer of drug in a like manner. The layering of the drug formulation and the osmopolymer layer can be fabricated by conventional two-layer press techniques. An analogous procedure may be followed for the preparation of the trilayered core. The compressed cores then may be coated with the inner wall material and the semipermeable wall material as described above.

Another manufacturing process that can be used comprises blending the powdered ingredients for each layer in a fluid bed granulator. After the powdered ingredients are dry blended in the granulator, a granulating fluid, for example, poly(vinylpyrrolidone) in water, is sprayed onto the powders. The coated powders are then dried in the granulator. This process granulates all the ingredients present therein while adding the granulating fluid. After the granules are dried, a lubricant, such as stearic acid or magnesium stearate, is mixed into the granulation using a blender e.g., V-blender or tote blender. The granules are then pressed in the manner described above.

The dosage form of the invention is provided with at least one exit 60. Exit 60 cooperates with the compressed core for the uniform release of drug from the dosage form. The exit can be provided during the manufacture of the dosage form or during drug delivery by the dosage form in a fluid environment of use.

Exit 60 may include an orifice that is formed or formable from a substance or polymer that erodes, dissolves or is leached from the outer wall to thereby form an exit orifice. The substance or polymer may include, for example, an erodible poly(glycolic) acid or poly(lactic) acid in the semipermeable wall; a gelatinous filament; a water-removable poly(vinyl alcohol); a leachable compound, such as a fluid removable pore-former selected from the group consisting of inorganic and organic salt, oxide and carbohydrate.

An exit, or a plurality of exits, can be formed by leaching a member selected from the group consisting of sorbitol, lactose, fructose, glucose, mannose, galactose, talose, sodium chloride, potassium chloride, sodium citrate and mannitol to provide a uniform-release dimensioned pore-exit orifice.

The exit can have any shape, such as round, triangular, square, elliptical and the like for the uniform metered dose release of a drug from the dosage form.

The dosage form can be constructed with one or more exits in spaced-apart relation or one or more surfaces of the dosage form.

Drilling, including mechanical and laser drilling, through the semipermeable wall can be used to form the exit orifice. Such exits and equipment for forming such exits are disclosed in U.S. Patents Nos. 3,916,899, by Theeuwes and Higuchi and in U.S. Patent No. 4,088,864, by Theeuwes, et al. It is presently preferred to utilize two exits of equal diameter.

Dosage forms of this invention exhibit sustained release of drug over a continuous time period that includes a prolonged time when drug is released at an ascending release rate as determined in a standard release rate assay such as that described herein. When administered to a subject, the dosage forms of the invention provide substantially ascending blood plasma drug concentrations in the subject that are less variable over a prolonged period of time than those obtained with immediate release dosage forms. When the dosage forms of this invention are administered on a continuous once-a-day basis, the dosage forms of the invention provide therapeutically effective ascending plasma drug concentrations while providing steady-state peak plasma drug concentrations that occur at a later time following dose administration and that exhibit a lesser magnitude than the steady-state peak plasma drug concentrations that occur following twice or three times a day administration of an immediate-release dosage form.

The practice of the foregoing methods of orally administering a dosage form to a subject once a day is preferred. Other disease states and conditions, which may be manifested or diagnosed as requiring an antipsychotic, may be treated with the paliperidone dosage forms and methods of the invention. In addition, other disease states and conditions which may or may not manifest in association with depression or anxiety, but which may be responsive to treatment with paliperidone may also be treated with the dosage forms and methods of the invention.

Preferred methods of manufacturing dosage forms of the present invention are generally described below. All percentages are weight percent unless otherwise noted.

### EXAMPLE 1

### Paliperidone Capsule Shaped Tablet, Trilayer 1.9 mg System

A dosage form adapted, designed and shaped as an osmotic drug delivery device is manufactured as follows: 100 g of paliperidone, 7345 g of polyethylene oxide with average molecular weight of 200,000, and 200 g of sodium chloride, USP are added to a fluid bed granulator bowl. Next a binder solution is prepared by dissolving 800 g of hydroxypropylmethyl cellulose identified as 2910 having an average viscosity of 5 cps in 9,200 g of water. The dry materials are fluid bed granulated by spraying with 6750 g of binder solution. Next, the wet granulation is dried in the granulator to an acceptable moisture content, and sized using by passing through a 7-mesh screen. Next, the granulation is transferred to a blender and mixed with 5 g of butylated hydroxytoluene as an antioxidant and lubricated with 50 g of stearic acid.

Next, a second drug compartment composition is prepared as follows: 280 g of paliperidone and 9165 g of polyethylene oxide with average molecular weight of 200,000 are added to a fluid bed granulator bowl. Next a binder solution is prepared by dissolving 800 g of hydroxypropylmethyl cellulose identified as 2910 having an average viscosity of 5 cps in 9,200 g of water. The dry materials are fluid bed granulated by spraying with 6750 g of binder solution. Next, the wet granulation is dried in the granulator to an acceptable moisture content, and sized using by passing through a 7-mesh screen. Next, the granulation is transferred to a blender and mixed with 5 g of butylated hydroxytoluene as an antioxidant and lubricated with 50 g of stearic acid.

Next, a push composition is prepared as follows: first, a binder solution is prepared. 15.6 kg of polyvinylpyrrolidone identified as K29-32 having an average molecular weight of 40,000 is dissolved in 104.4 kg of water. Then, 24 kg of sodium chloride and 1.2 kg of ferric oxide are sized using a Quadro Comil with a 21-mesh screen. Then, the screened materials and 88.44 kg of Polyethylene oxide (approximately 7,000,000 molecular weight) are added to a fluid bed granulator bowl. The dry materials are fluidized and mixed while 46.2 kg of binder solution is sprayed from 3 nozzles onto the powder. The granulation is dried in the fluid-bed chamber to an acceptable moisture level. The coated granules are sized using a Fluid Air mill with a 7-mesh screen. The granulation is transferred to a tote tumbler, mixed with 15 g of butylated hydroxytoluene and lubricated with 294 g magnesium stearate.

Next, the pallperidone drug compositions for the first and the second compartments and the push composition are compressed into trilayer tablets. First, 50 mg of the paliperidone compartment one composition is added to the die cavity and pre-compressed, then 50 mg of the paliperidone compartment two composition is added to the die cavity and pre-compressed, then 110 mg of the push composition is added and the layers are pressed into a 3/16" (4.8mm) diameter longitudinal, deep concave, trilayer arrangement.

The trilayered arrangements are coated with a subcoat laminate. The wall forming composition comprises 70% hydroxypropyl cellulose identified as EF, having an average molecular weight of 80,000 and 30% of polyvinylpyrrolidone identified as K29-32 having an average molecular weight of 40,000. The wall-forming composition is dissolved in anhydrous ethyl alcohol, to make an 8% solids solution. The wall-forming composition is sprayed onto and around the bilayered arrangements in a pan coater until approximately 20 mg of laminate is applied to each tablet.

The trilayered arrangements are coated with a semi-permeable wall. The wall forming composition comprises 99% cellulose acetate having a 39.8% acetyl content and 1% polyethylene glycol comprising a 3.350 viscosity-average molecular weight The wall-forming composition is dissolved in an acetone:water (95:5 wt:wt) co solvent to make a 5% solids solution. The wall-forming composition is sprayed onto and around the bilayered arrangements in a pan coater until approximately 45 mg of membrane is applied to each tablet.

Next, two 25 mil (0.6 mm) exit passageways are laser drilled through the semi-permeable wall to connect the drug layer with the exterior of the dosage system. The residual solvent is removed by drying for 144 hours as 45 C. and 45% humidity. After drilling, the osmotic systems are dried for 4 hours at 45 C. to remove excess moisture.

The dosage form produced by this manufacture is designed to deliver 1.9 mg of paliperidone in an ascending delivery pattern from two drug-containing cores. First core contains 1% paliperidone, 73.45% polyethylene oxide possessing a 200,000 molecular weight, 20% sodium chloride, USP, 5% hydroxypropylmethyl cellulose having an average viscosity of 5 cps, 0.05% butylated hydroxytoluene, and 0.5% stearic acid. Second drug core contains 2.8% paliperidone, 91.65% polyethylene oxide possessing a 200,000 molecular weight, 5% hydroxypropylmethyl cellulose having an average viscosity of 5 cps, 0.05% butylated hydroxytoluene, and 0.5% stearic acid. The push composition is comprised 73.7% polyethylene oxide comprising a 7,000,000 molecular weight, 20% sodium chloride, 5% polyvinylpyrrolidone possessing an average molecular weight of 40,000, 1% ferric oxide, 0.05% butylated hydroxytoluene, and 0.25% magnesium stearate. The semi permeable wall is comprised of 99% cellulose acetate of 39.8% acetyl content and 1% polyethylene glycol. The dosage form comprises two passageways, 25 mils (0.6 mm) on the center of the drug side.

### Example 2

### Paliperidone Capsule Shaped Tablet, Trilayer 0.5 mg System

A dosage form adapted, designed and shaped as an osmotic drug delivery device is manufactured as follows: 25 g of paliperidone, 7420 g of polyethylene oxide with average molecular weight of 200,000, and 200 g of sodium chloride, USP are added to a fluid bed granulator bowl. Next a binder solution is prepared by dissolving 800 g of hydroxypropylmethyl cellulose identified as 2910 having an average viscosity of 5 cps in 9,200 g of water. The dry materials are fluid bed granulated by spraying with 6750 g of binder solution. Next, the wet granulation is dried in the granulator to an acceptable moisture content, and sized using by passing through a 7-mesh screen. Next, the granulation is transferred to a blender and mixed with 5 g of butylated hydroxytoluene as an antioxidant and lubricated with 50 g of stearic acid.

Next, a second drug compartment composition is prepared as follows: 70 g of paliperidone and 9375 g of polyethylene oxide with average molecular weight of 200,000 are added to a fluid bed granulator bowl. Next a binder solution is prepared by dissolving 800 g of hydroxypropylmethyl cellulose identified as 2910 having an average viscosity of 5 cps in 9,200 g of water. The dry materials are fluid bed granulated by spraying with 6750 g of binder solution. Next, the wet granulation is dried in the granulator to an acceptable moisture content, and sized using by passing through a 7-mesh screen. Next, the granulation is transferred to a blender and mixed with 5 g of butylated hydroxytoluene as an antioxidant and lubricated with 50 g of stearic acid.

Next, a push composition is prepared as follows: first, a binder solution is prepared. 15.6 kg of polyvinylpyrrolidone identified as K29-32 having an average molecular weight of 40,000 is dissolved in 104.4 kg of water. Then, 24 kg of sodium chloride and 1.2 kg of ferric oxide are sized using a Quadro Comil with a 21-mesh screen. Then, the screened materials and 88.44 kg of Polyethylene oxide (approximately 7,000,000 molecular weight) are added to a fluid bed granulator bowl. The dry materials are fluidized and mixed while 46.2 kg of binder solution is sprayed from 3 nozzles onto the powder. The granulation is dried in the fluid-bed chamber to an acceptable moisture level. The coated granules are sized using a Fluid Air mill with a 7-mesh screen. The granulation is transferred to a tote tumbler, mixed with 15 g of butylated hydroxytoluene and lubricated with 294 g magnesium stearate.

Next, the paliperidone drug compositions for the first and the second compartments and the push composition are compressed into trilayer tablets. First, 50 mg of the paliperidone compartment one composition is added to the die cavity and pre-compressed, then 50 mg of the paliperidone compartment two composition is added to the die cavity and pre-compressed, then 110 mg of the push composition is added and the layers are pressed into a 3/16" (4.8mm) diameter longitudinal, deep concave, trilayer arrangement.

The trilayered arrangements are coated with a subcoat laminate. The wall forming composition comprises 70% hydroxypropyl cellulose identified as EF, having an average molecular weight of 80,000 and 30% of polyvinylpyrrolidone identified as K29-32 having an average molecular weight of 40,000. The wall-forming composition is dissolved in anhydrous ethyl alcohol, to make an 8% solids solution, The wall-forming composition is sprayed onto and around the bilayered arrangements in a pan coater until approximately 20 mg of laminate is applied to each tablet.

The trilayered arrangements are coated with a semi-permeable wall. The wall forming composition comprises 99% cellulose acetate having a 39.8% acetyl content and 1% polyethylene glycol comprising a 3.350 viscosity-average molecular weight The wall-forming composition is dissolved in an acetone:water (95:5 wt:wt) co solvent to make a 5% solids solution. The wall-forming composition is sprayed onto and around the bilayered arrangements in a pan coater until approximately 39 mg of membrane is applied to each tablet.

Next, two 25 mil (0.6 mm) exit passageways are laser drilled through the semi-permeable wall to connect the drug layer with the exterior of the dosage system. The residual solvent is removed by drying for 144 hours as 45 C. and 45% humidity. After drilling, the osmotic systems are dried for 4 hours at 45 C to remove excess moisture.

The dosage form produced by this manufacture is designed to deliver 0.25 mg of paliperidone in an ascending delivery pattern from two drug-containing cores. First core contains 0.25% paliperidone, 74.20% polyethylene oxide possessing a 200,000 molecular weight, 20% sodium chloride, USP, 5% hydroxypropylmethyl cellulose having an average viscosity of 5 cps, 0.05% butylated hydroxytoluene, and 0.5% stearic acid. Second drug core contains 0.7% paliperidone, 93.75% polyethylene oxide possessing a 200,000 molecular weight, 5% hydroxypropylmethyl cellulose having an average viscosity of 5 cps, 0.05% butylated hydroxytoluene, and 0.5% stearic acid. The push composition is comprised 73.7% polyethylene oxide comprising a 7,000,000 molecular weight, 20% sodium chloride, 5% polyvinyl pyrrolidone possessing an average molecular weight of 40,000, 1% ferric oxide, 0.05% butylated hydroxytoluene, and 0.25% magnesium stearate. The semi permeable wall is comprised of 99% cellulose acetate of 39.8% acetyl content and 1% polyethylene glycol. The dosage form comprises two passageways, 25 mils (0.6 mm) on the center of the drug side.

## Claims

1. A dosage form, comprising:
two or more layers, said two or more layers comprising a first layer and a second layer, said first layer comprises paliperidone, risperidone or a pharmaceutically acceptable salt thereof, said second layer comprises a polymer; an outer wall surrounding said two or more layers; and an orifice in said outer wall.

2. The dosage form of claim 1, wherein said first layer comprises an osmagent.

3. The dosage form of claim 2, wherein said osmagent is sodium chloride.

4. The dosage form of claim 2 or 3, wherein said osmagent is present at least 20% by weight in said first layer.

5. The dosage form of any one of claims 1 to 4, wherein said first layer comprises paliperidone or a pharmaceutically acceptable salt thereof and said second layer further comprises paliperidone or a pharmaceutically acceptable salt thereof, and wherein the ratio of the amount of paliperidone or a pharmaceutically acceptable salt thereof in said first layer to the amount of paliperidone or a pharmaceutically acceptable salt thereof in said second layer is less than 1.0.

6. The dosage form of claim 5, wherein the ratio of the amount of paliperidone or a pharmaceutically acceptable salt thereof in said first layer to the amount of paliperidone or pharmaceutically acceptable salt thereof in said second layer is less than 0.44.

7. The dosage form of claim 5, wherein the ratio of the amount of paliperidone or a pharmaceutically acceptable salt thereof in said first layer to the amount of paliperidone or pharmaceutically acceptable salt thereof in said second layer is less than 0.33.

8. The dosage form of any one of claims 1 to 7, further comprising a subcoat surrounding said first layer and said second layer and within said outer wall.

9. The dosage form of claim 8, wherein said subcoat comprises a hydroxyalkylcellulose polymer having a molecular weight from about 8,500 to about 4,000,000.

10. Use of a pharmaceutically effective amount of paliperidone, risperidone or a pharmaceutically acceptable salt thereof in the manufacture of a dosage form according to any one of claims 1 to 9 for reducing the side effects associated with high blood plasma concentration of antipsychotic agents, wherein said dosage form maintains an ascending release rate of said paliperidone, risperidone or pharmaceutically acceptable salt thereof over a prolonged period of time.

11. Use according to claim 10, wherein the side effects are selected from the group consisting of anxiety, somnolence, dizziness, constipation, and extrapyramidal symptoms.

12. Use according to claim 10 or 11, wherein said ascending release rate is maintained for 10 to 24 hours after administration.

13. Use according to claim 10 or 11, wherein said ascending release rate is maintained for 16 to 22 hours after administration.

14. Use according to claim 10 or 11, wherein said ascending release rate is maintained for 18 to 21 hours after administration.

15. Use according to any one of claims 10 or 14, wherein Cₘₐₓ occurs at about 14 hours after administration.

16. Use according to any one of claims 10 or 14, wherein Cₘₐₓ occurs at between 16 and 22 hours after administration.

17. Use according to any one of claims 10 or 14, wherein Cₘₐₓ occurs at between 18 hours and 21 hours after administration.

18. Use according to any one of claims 10 to 17, wherein T₉₀ occurs at between 18 hours and 22 hours after administration.

19. Use according to any of claims 10 to 18, wherein said dosage form is suitable for administering once a day.

## Patentansprüche

1. Dosierungsform, umfassend zwei oder mehr Schichten, wobei die zwei oder mehr Schichten eine erste Schicht und eine zweite Schicht umfassen, wobei die erste Schicht Paliperidon, Risperidon oder ein pharmazeutisch verträgliches Salz davon umfasst, wobei die zweite Schicht ein Polymer umfasst; eine die zwei oder mehr Schichten umgebende Außenwand; und eine Öffnung in der Außenwand.

2. Dosierungsform nach Anspruch 1, wobei die erste Schicht ein Osmagens umfasst.

3. Dosierungsform nach Anspruch 2, wobei das Osmagens Natriumchlorid ist.

4. Dosierungsform nach Anspruch 1 oder 2, wobei das Osmagens mit mindestens 20 Gew.-% in der ersten Schicht vorliegt.

5. Dosierungsform nach einem der Ansprüche 1 bis 4, wobei die erste Schicht Paliperidon oder ein pharmazeutisch verträgliches Salz davon und die zweite Schicht ferner Paliperidon oder ein pharmazeutisch verträgliches Salz davon umfasst und wobei das Verhältnis der Menge an Paliperidon oder eines pharmazeutisch verträglichen Salzes davon in der ersten Schicht zu der Menge an Paliperidon oder eines pharmazeutisch verträglichen Salzes davon in der zweiten Schicht weniger als 1,0 beträgt.

6. Dosierungsform nach Anspruch 5, wobei das Verhältnis der Menge an Paliperidon oder eines pharmazeutisch verträglichen Salzes davon in der ersten Schicht zu der Menge an Paliperidon oder eines pharmazeutisch verträglichen Salzes davon in der zweiten Schicht weniger als 0,44 beträgt.

7. Dosierungsform nach Anspruch 5, wobei das Verhältnis der Menge an Paliperidon oder eines pharmazeutisch verträglichen Salzes davon in der ersten Schicht zu der Menge an Paliperidon oder eines pharmazeutisch verträglichen Salzes davon in der zweiten Schicht weniger als 0,33 beträgt.

8. Dosierungsform nach einem der Ansprüche 1 bis 7, ferner umfassend eine Grundschicht, die die erste Schicht und die zweite Schicht umgibt und in der Außenwand vorliegt.

9. Dosierungsform nach Anspruch 8, wobei die Grundschicht ein Hydroxyalkylcellulosepolymer mit einem Molekulargewicht von etwa 8.500 bis etwa 4.000.000 umfasst.

10. Verwendung einer pharmazeutisch wirksamen Menge Paliperidon, Risperidon oder eines pharmazeutisch verträglichen Salzes davon bei der Herstellung einer Dosierungsform nach einem der Ansprüche 1 bis 9 zum Reduzieren der mit einer hohen Blutplasmakonzentration antipsychotischer Mittel verbundenen Nebenwirkungen, wobei die Dosierungsform eine ansteigende Freisetzungsrate des Paliperidons, Risperidons oder pharmazeutisch verträglichen Salzes davon über eine längere Zeitdauer aufrecht erhält.

11. Verwendung nach Anspruch 10, wobei die Nebenwirkungen ausgewählt sind aus der Gruppe, bestehend aus Beklemmung, Schläfrigkeit, Schwindel, Verstopfung und extrapyramidalen Symptomen.

12. Verwendung nach Anspruch 10 oder 11, wobei die ansteigende Freisetzungsrate für eine Dauer von 10 bis 24 Stunden nach Verabreichung aufrechterhalten wird.

13. Verwendung nach Anspruch 10 oder 11, wobei die ansteigende Freisetzungsrate für eine Dauer von 16 bis 22 Stunden nach Verabreichung aufrechterhalten wird.

14. Verwendung nach Anspruch 10 oder 11, wobei die ansteigende Freisetzungsrate für eine Dauer von 18 bis 21 Stunden nach Verabreichung aufrechterhalten wird.

15. Verwendung nach einem der Ansprüche 10 oder 14, wobei Cₘₐₓ etwa 14 Stunden nach verabreichung eintritt.

16. Verwendung nach einem der Ansprüche 10 oder 14, wobei Cₘₐₓ zwischen 16 und 22 Stunden nach Verabreichung eintritt.

17. Verwendung nach einem der Ansprüche 10 oder 14, wobei Cₘₐₓ zwischen 18 und 21 Stunden nach Verabreichung eintritt.

18. Verwendung nach einem der Ansprüche 10 bis 17, wobei T₂₀ zwischen 18 und 22 Stunden nach Verabreichung eintritt.

19. Verwendung nach einem der Ansprüche 10 bis 18, wobei die Dosierungsform zur einmal täglichen Verabreichung geeignet ist.

## Revendications

1. - Forme posologique, comprenant :
- deux couches ou plus, lesdites deux couches ou plus comprenant une première couche et une seconde couche, ladite première couche comprend de la palipéridone, de la rispéridone ou un sel pharmaceutiquement acceptable de celles-ci, ladite seconde couche comprend un polymère ;
- une paroi externe entourant lesdites deux couches ou plus ; et
- un orifice dans ladite paroi externe.

2. - Forme posologique selon la revendication 1, dans laquelle ladite première couche comprend un osmagent.

3. - Forme posologique selon la revendication 2, dans laquelle ledit osmoagent est le chlorure de sodium.

4. - Forme posologique selon l'une des revendications 2 ou 3, dans laquelle ledit osmagent est présent à au moins 20% en poids dans ladite première couche.

5. - Forme posologique selon l'une quelconque des revendications 1 à 4, dans laquelle ladite première couche comprend de la palipéridone ou un sel pharmaceutiquement acceptable de celle-ci et ladite seconde couche comprend en outre de la palipéridone ou un sel pharmaceutiquement acceptable de celle-ci, et dans laquelle le rapport de la quantité de palipéridone ou d'un sel pharmaceutiquement acceptable de celle-ci dans ladite première couche à la quantité de palipéridone ou d'un sel pharmaceutiquement acceptable de celle-ci dans ladite seconde couche est inférieur à 1,0.

6. - Forme posologique selon la revendication 5, dans laquelle le rapport de la quantité de palipéridone ou d'un sel pharmaceutiquement acceptable de celle-ci dans ladite première couche à la quantité de palipéridone ou d'un sel pharmaceutiquement acceptable de celle-ci dans ladite seconde couche est inférieur à 0,44.

7. - Forme posologique selon la revendication 5, dans laquelle le rapport de la quantité de palipéridone ou d'un sel pharmaceutiquement acceptable de celle-ci dans ladite première couche à la quantité de palipéridone ou d'un sel pharmaceutiquement acceptable de celle-ci dans ladite seconde couche est inférieur à 0,33.

8. - Forme posologique selon l'une quelconque des revendications 1 à 7, comprenant en outre une sous-couche entourant ladite première couche et ladite seconde couche et à l'intérieur de ladite paroi externe.

9. - Forme posologique selon la revendication 8, dans laquelle ladite sous-couche comprend un polymère d'hydroxyalkylcellulose ayant une masse moléculaire d'environ 8 500 à environ 4 000 000.

10. - Utilisation d'une quantité pharmaceutiquement efficace de palipéridone, de rispéridone ou d'un sel pharmaceutiquement acceptable de celles-ci dans la fabrication d'une forme posologique telle que définie à l'une quelconque des revendications 1 à 9, pour réduire les effets secondaires associés à une concentration élevée dans le plasma sanguin d'agents antipsychotiques, dans laquelle ladite forme posologique maintient une vitesse de libération croissante de ladite palipéridone, rispéridone ou d'un sel pharmaceutiquement acceptable de celles-ci sur une période de temps prolongée.

11. - Utilisation selon la revendication 10, dans laquelle les effets secondaires sont choisis dans le groupe constitué par l'anxiété, la somnolence, l'état vertigineux, la constipation et les symptômes extrapyramidaux.

12. - Utilisation selon l'une des revendications 10 ou 11, dans laquelle ladite vitesse de libération croissante est maintenue pendant 10 à 24 heures après administration.

13. - Utilisation selon l'une des revendications 10 ou 11, dans laquelle ladite vitesse de libération croissante est maintenue pendant 16 à 22 heures après administration.

14. - Utilisation selon l'une des revendications 10 ou 11, dans laquelle ladite vitesse de libération croissante est maintenue pendant 18 à 21 heures après administration.

15. - Utilisation selon l'une quelconque des revendications 10 ou 14, dans laquelle Cₘₐₓ se produit à environ 14 heures après administration.

16. - Utilisation selon l'une quelconque des revendications 10 ou 14, dans laquelle Cₘₐₓ se produit à entre 16 et 22 heures après administration.

17. - Utilisation selon l'une quelconque des revendications 10 ou 14, dans laquelle Cₘₐₓ se produit à entre 18 et 21 heures après administration.

18. - Utilisation selon l'une quelconque des revendications 10 à 17, dans laquelle T₉₀ se produit à entre 18 et 22 heures après administration.

19. - Utilisation selon l'une quelconque des revendications 10 à 18, dans laquelle ladite forme posologique est appropriée pour une administration une fois par jour.
